# EUROPEAN PATENT APPLICATION

(11) **EP 1 952 776 A1**
(43) Date of publication of application: **06.08.2008**
(21) Application number: 08150862.4
(22) Date of filing: 31.01.2008
(51) Int. Cl.: A61B 17/80, A61B 17/72

(54) **Implant for fixing two fragments of bone together in osteotomy procedures**

(30) Priority: 01.02.2007 IT VR20070017
(71) Applicant: Lodola, Giuseppe, 37060 Alpo di Villafranca (VR) (IT)
(72) Inventor: Lodola, Giuseppe, 37060 Alpo di Villafranca (VR) (IT)
(74) Representative: Petraz, Gilberto Luigi

(57) **Abstract**

An implant for fixing two bone fragments together, following the axial correction of the metatarsal head with respect to the metatarsal segment in hallux valgus deformities, in osteotomy procedures in general, consists of a cylindrical cross-section curved pin (10), with a tip (15) of the curvature (14) and a proximal part in the shape of a paddle (16) equipped with a central hole flared at the surface and threaded in its lower portion, designed to accommodate a flat-head screw (12) which prevents dangerous protruberances into the soft tissues.

According to another embodiment of the modular implant, two components are foreseen, an L-shaped plate (23) angled at approximately 3° with respect to the longitudinal axis of the assembled implant, and a cone-shaped component (24) with a curved tip.

## Description

### TECHNICAL FIELD

This invention refers to an implant for fixing two fragments of bone together following the axial correction of the metatarsal head with respect to the metatarsal segment in hallux valgus, in osteotomy procedures.

More specifically, the main objective of this invention is to fix the two bone fragments, following osteotomy, of the first metatarsal, in hallux valgus, and to adjust the lateralization, plantarization and orientation on the frontal and sagittal planes (alpha and omega), preventing mobilization between the two fragments during bone consolidation and allowing bone union of the two fragments without displacement.

The use is also foreseen of a standard anatomical conformation modular type endo-osseous system, that is to say with the possibility, by means of a special instrument, of further lateralizing the two bone segments during surgery until anatomical alignment is achieved. This implies blocking the modular endo-osseous implant in the required anatomical position and preventing mobilization between the two bone segments during consolidation without any displacement and in the required position.

This invention does in fact have the dual aim of fixing the two bone segments following distal linear osteotomy of the first metatarsal, in hallux valgus, and also if anatomically necessary, of increasing the lateralization, adjusting the plantarization and the orientation on the frontal and sagittal planes, in addition to preventing mobilization between the two bone fragments, thus allowing bone union without displacement.

The device according to the invention, which substantially consists of a curved pin implant specifically mounted on the positioning guide and blocked by a threaded cannula which also acts as a perforation guide for the preparation of the flat-head screw housing, is sunk in the medullary canal of the metatarsal bone segment as far as its base.

The device according to the invention also foresees the use of a modular implant solution which includes two components: a cone-shaped component with a curved tip and an L-shaped plate with a hole to accommodate a flat-head screw on one side, two micro holes on the underside for pre-stabilization and a micro grub screw for blocking the two modular components on completion of translation. The system also includes an instrument for positioning the endo-osseous implant and a translator instrument to be fitted to the modular endo-osseous implant, with a thrust system to produce the millimetric translation.

This invention can be applied in the orthopedic field and in particular in osteotomy performed in cases of hallux valgus.

### BACKGROUND ART

It is known that the hallux valgus deformity is characterised by the progressive lateral deviation of the great toe and by the medial deviation of the first metatarsal.

Hallux valgus is the most frequent deformity of the foot and is usually not an isolated event, but is part of more complex alterations of the entire foot, of which it is only the most evident aspect.

Congenital forms are rare, while acquired forms, frequent especially in females, are the result of complex alterations of the static and dynamic equilibrium of the foot (in particular of the subtalar articulation).

Although less important, the mechanical stimuli caused to an Egyptian morphotype foot (great toe longer than the other toes) by shoes that are too pointed or that have an excessively high heel should not be ignored.

Once the deformity has started, it is worsened by the traction of the muscles attached to the phalanges of the great toe (extensor hallucis, flexor hallucis and adductor hallucis).

Gradually but inexorably the base of the first phalanx deviates outwards and the head of the metatarsal moves inwards.

A reactive borsitis forms in the overlying, soft tissues, which can easily become inflamed.

The treatment of hallux valgus depends on various parameters which should be carefully considered: age of the patient, clinical conditions, with particular attention to the vascular, neurologic and metabolic systems, requirements connected with the footwear worn and physical activity, and finally radiographic parameters.

There is a wide range of methods for the care and correction of these deformities, although surgical correction offers the best results, these being, in most cases, definitive. Surgery is also performed under local anesthesia (anesthesia limited to the leg) and postoperative recovery is very fast.

By way of example, the following types of surgery can be mentioned: Chevron's osteotomy, Akin's osteotomy, oblique distal metatarsal osteotomy, proximal osteotomy with distal soft tissue balancing, diaphyseal osteotomy of the first metatarsal (SCARF), Keller's osteotomy, Lelievre's osteotomy, Villadot's osteotomy, Regnauld's osteotomy, metatarsal-phalangeal arthrodesis, and others.

SCARF osteotomy is performed with an appropriate oscillating saw, at the level of the first metatarsal with a Z design. This osteotomy allows a correction in the three planes of the space of the metatarsal head and is therefore widely applied in the treatment of hallux valgus. In particular, it allows correction of the intermetatarsal angle by laterally displacing the head and correction of the articular angle by appropriately turning the articular surface; it is also possible, when indicated, to shorten or lengthen the metatarsal and to displace the head in a plantar or dorsal direction. Once the deformity has been corrected, this osteotomy is fixed by means of two titanium screws. It is often associated with an osteotomy at the base of the proximal phalange according to Akin. The latter is fixed with a steel staple.

AUSTIN's osteotomy is also performed with an appropriate oscillating saw, at the level of the neck of the first metatarsal, with a distal apex V design which already provides a certain stability. The cephalic bone fragment is moved laterally and often also in a plantar direction to correct the deformity of the first metatarsal, which is at the base of the hallux valgus. Finally, the osteotomy is fixed with a screw.

Another case concerns osteotomy according to Bösch, which is performed using a metal wire, following a small medial incision at the head of the first metatarsal. The head is then displaced laterally and stabilized with a wire that exits from the apex of the great toe. The wire is removed 30 days after the operation.

In all the situations described above and in other similar surgical techniques, problems have been encountered regarding on one hand the practical difficulties involved in carrying out the operation and on the other the difficulties in patient rehabilitation.

These difficulties are due to the lack of suitable means of connection between the bone fragments to be joined, and in particular to the lack of a suitable connector that makes the insertion phases easier and also ensures faster healing times, something that would greatly benefit the patient and make the rehabilitation more acceptable.

### DESCRIPTION OF THE INVENTION

This invention proposes to provide an implant for fixing two bone fragments together, able to eliminate or at least reduce the problems described above.

The invention also proposes to provide an implant for fixing two bone fragments together, in particular for osteotomy operations for the correction of hallux valgus, which is easy to produce so as to be economically inexpensive from the production point of view, and thus possible to produce on a large scale.

It is also foreseen that the endo-osseous system according to the invention can be the modular type, that is to say with a standard anatomical conformation, but with the possibility, by means of a special instrument, of further lateralizing the two bone segments during the operation until anatomical alignment is achieved, and then blocking the modular endo-osseous implant in the required anatomical position, preventing mobilization between the two bone segments during bone consolidation without displacement and in the required position.

This is achieved by means of an implant for fixing two bone fragments together in osteotomy procedures for the correction of hallux valgus, the features of which are described in the main claim.

The dependent claims of the solution in question describe advantageous embodiments of the invention.

The main advantages of this solution, in addition to those consequent to the construction simplicity, concern first of all the fact of using a particularly shaped connector, which makes it possible to fix the two bone fragments following osteotomy, of the first metatarsal, in hallux valgus, and to adjust the lateralization, plantarization and orientation on the frontal and sagittal planes (alpha and omega), preventing mobilization between the two fragments during bone consolidation and allowing bone union of the two fragments without displacement.

The device according to the invention thus substantially consists of a particularly shaped curved pin and a screw.

The curved pin with a bevelled tip is inserted in the medullary canal of the metatarsal segment, with the tip of the curve resting on the external medial cortex and the proximal paddle-shaped part resting on the previously osteotomized head of the metatarsal, automatically lateralizing it.

The paddle-shaped part presents a threaded hole designed to accommodate a flat-head screw for the fixing and definitive stabilization of the metatarsal head to the metatarsal bone segment. Below the flared part of the head, the screw is equipped with mechanical threading, designed to stabilize it when screwed into the threaded hole of the curved pin, and bone type threading designed to pierce the metatarsal head and form a one-piece body between the implant and the metatarsal component.

The aim of the invention is to perform a surgical procedure easily and precisely, to adjust the bone fragment of the first metatarsal with respect to the other metatarsal fragment, following osteotomy, and to provide stability to the implant, in relation to the two metatarsal bone segments.

The use is also foreseen of a standard anatomical conformation modular type endo-osseous system, that is to say with the possibility, by means of a special instrument, of further lateralizing the two bone segments during surgery until anatomical alignment is achieved. This implies blocking the modular endo-osseous implant in the required anatomical position and preventing mobilization between the two bone segments during consolidation without any displacement. and in the required position.

The modular implant according to the invention thus consists of a two-component modular endo-osseous implant and an instrument designed to perform a gradual correction of the translation. The assembled modular endo-osseous implant, consisting of a cone-shaped part with a curved tip and a knurled base attached to an L-shaped plate with a knurled base, is inserted in the medullary canal of the metatarsal segment with the tip of the curve resting on the external medial cortex and the L-shaped plate consequently resting on the previously osteotomized head of the metatarsal, automatically lateralizing it. The additional correction of the lateralization of the metatarsal head with respect to the metatarsal segment is provided by the progressive lateral translation between the two modular components, until the correct translation is achieved. Blocking of the two modular components is ensured by a blocking screw. The L-shaped plate presents a flared hole with mechanical type threading designed to accommodate a flat-head screw for the fixing and definitive stabilization between the metatarsal head and the metatarsal bone segment. The screw is equipped with bone type threading, with a pyramidal tip, and mechanical type threading below the flared head, designed to stabilize the L-shaped plate when screwed into the threaded hole of the curved pin, to prevent any expulsion due to thrust forces during walking. The aim of the invention is to perform a surgical procedure easily and precisely, using a surgical device and its relative instrument, in order to adjust the correct displacement of the metatarsal head after distal osteotomy, according to the anatomical requirements, and to provide maximum stability to the implant, in relation to the two bone segments until consolidation is achieved.

### DESCRIPTION OF THE DRAWINGS

Other features and advantages of the invention will become evident on reading the description given below of one embodiment of the invention, provided as a non-binding example, with the help of the accompanying drawings, in which:

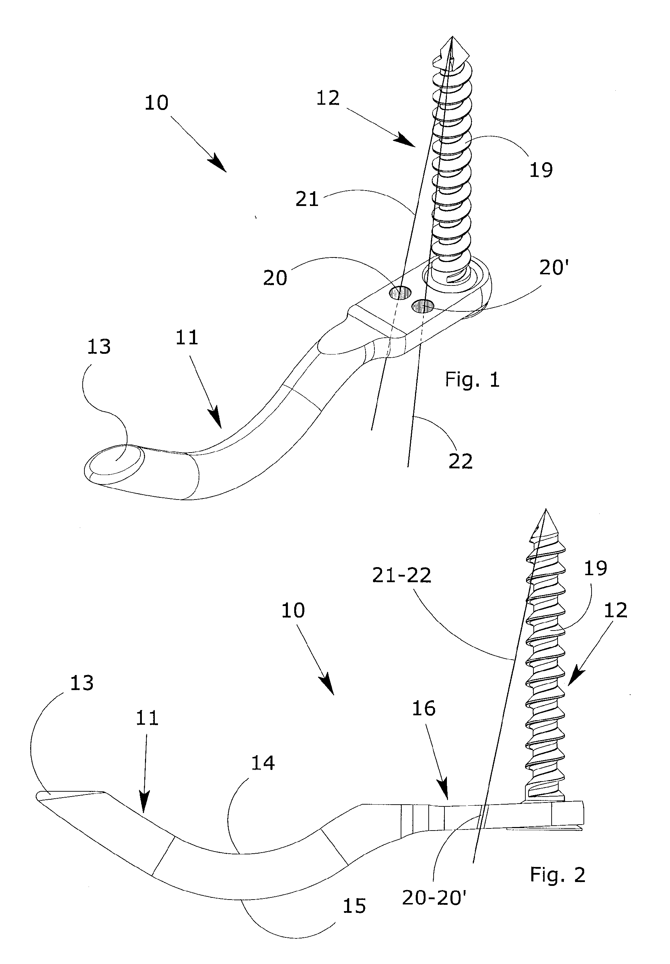

- figure 1 represents a schematic and prospective view of a pin according to the invention in its entirety;
- figure 2 is a schematic side view of a pin according to the invention;
- figure 3 is a schematic longitudinal cross-section side view of a pin according to the invention;
- figure 4 is a schematic plan view of a pin according to the invention;
- figure 5 shows a schematic side view of the same pin;
- figure 6 is a schematic view of the screw for fixing the pin to the bone structure;
- figures 7 and 8 show schematic plan and side views of the pin of the previous figures;
- figure 9 is a schematic view of a pin according to the invention in an example applied to the bone structure;
- figures 10 to 13 are schematic views taken from various angles showing the L-shaped plate forming part of the modular endo-osseous implant;
- figures 14 to 17 are schematic views showing the cone-shaped component with a curved tip forming part of the modular endo-osseous implant;
- figures 18 and 19 show a side and prospective view of the modular endo-osseous implant consisting of a cone-shaped component with a curved tip, an L-shaped plate and the stabilization screw fitted;

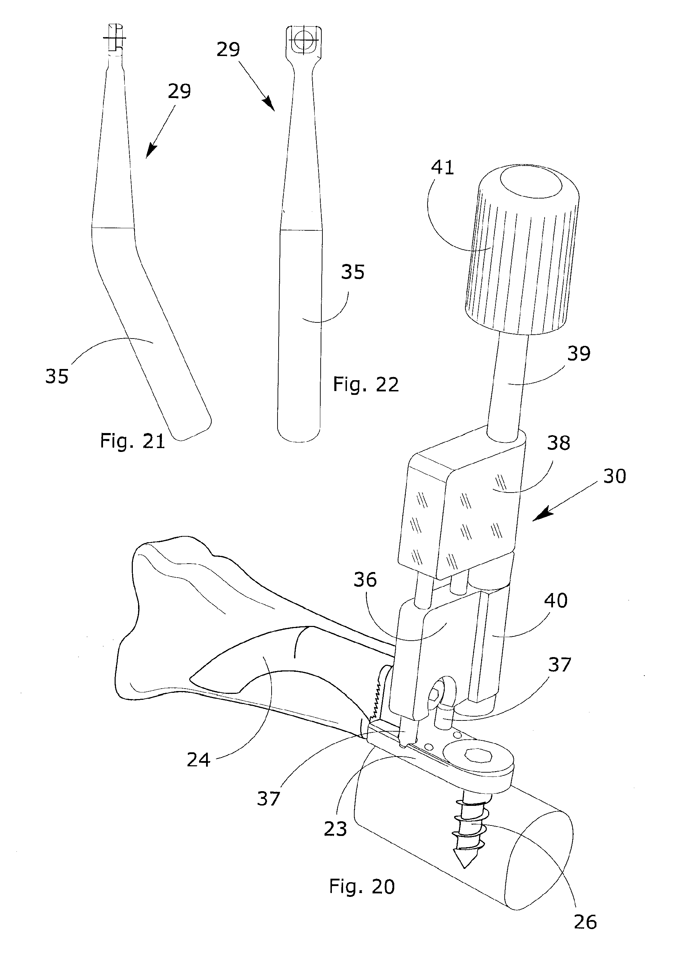

- figure 20 is a schematic view of the modular endo-osseous implant according to the invention, consisting of a cone-shaped component with a curved tip, an L-shaped plate and a stabilization screw fitted, equipped with a translator instrument, consisting of a thrust block, with two pins underneath, a thrust rod passing through medially and a threaded rod at the distal level which when appropriately screwed by means of the proximal knob, produces the translation effect between the two components of the endo-osseous implant, with the correct alignment between the metatarsal head and the metatarsal bone component of the first metatarsal in relation to the anatomical requirement;
- figures 21 and 22 show views of the instrument for positioning the endo-osseous implant.

### DESCRIPTION OF ONE EMBODIMENT OF THE INVENTION

Referring to the accompanying figures, the reference number 10 indicates overall an implant for fixing two bone fragments together, following the axial correction of the metatarsal head with respect to the metatarsal segment in hallux valgus, in osteotomy procedures.

This implant substantially consists of a curved pin 11 and a screw 12.

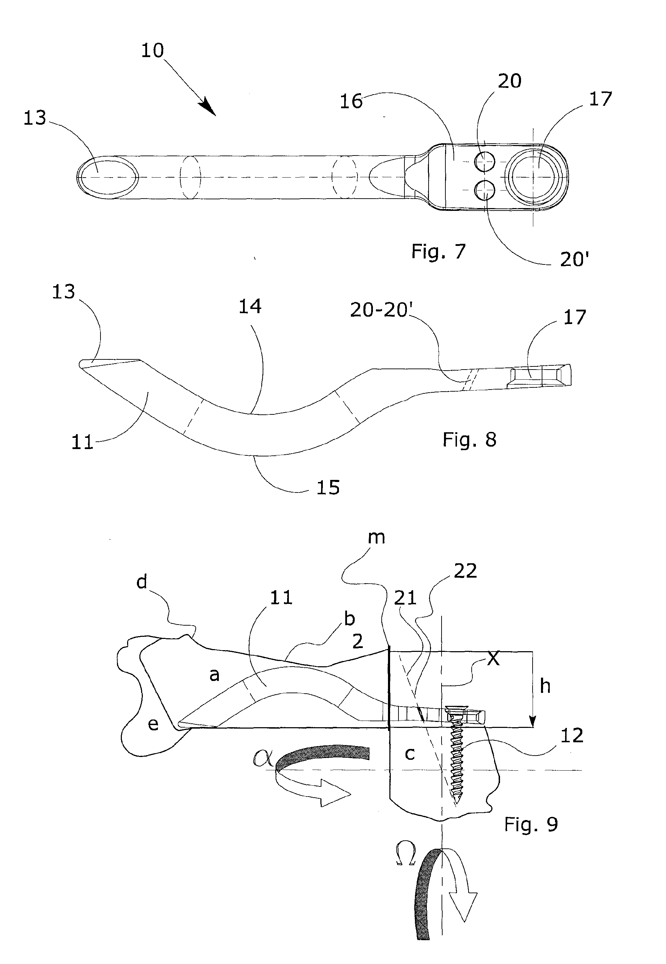

The curved pin 11 presents a bevelled tip 13, which, as can been in figure 9, represents the portion to be inserted in the medullary canal (a) of the metatarsal segment (d) (see diagram in figure 9).

The pin 11 also presents a curved section 14, the end 15 of which is designed to rest against the external medial cortex (b).

The pin 10 presents a proximal paddle-shaped part 16, which rests against the previously osteotomized metatarsal head (c), automatically lateralizing it (h).

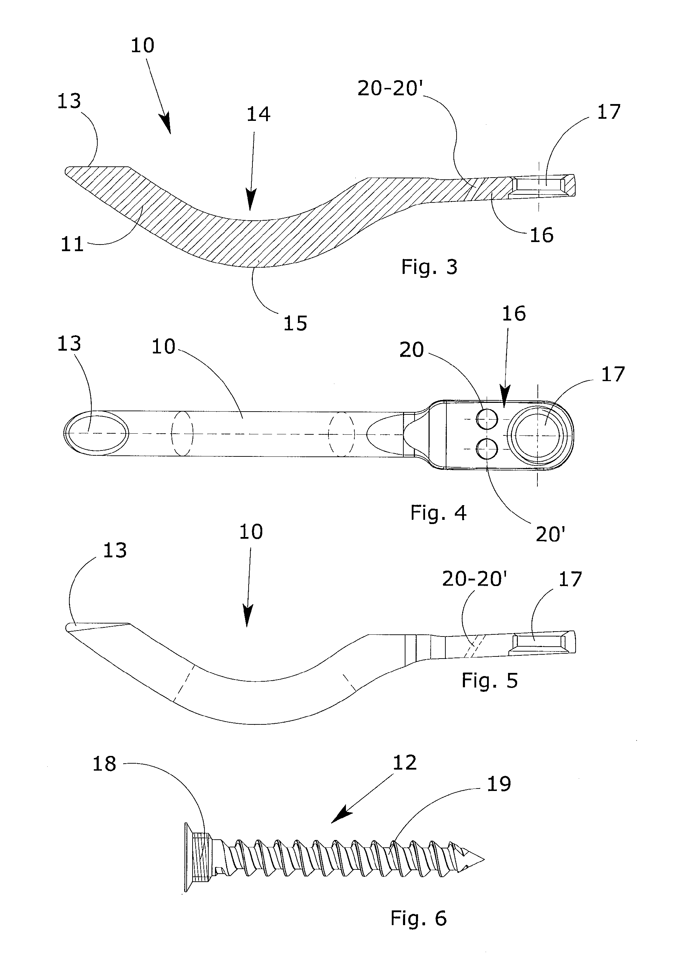

The paddle-shaped part 16 presents a threaded hole 17 (figs. 3, 4 and 5) designed to accommodate a flat-head screw 12 which prevents dangerous protruberances on the soft tissues for the definitive fixing and mobilization of the metatarsal head (c) to the metatarsal bone segment (d).

The screw, which a flared head 18, is equipped with mechanical type threading close to the head designed to stabilize it by screwing into the threaded hole 17, with the curved pin, while the shank presents bone type threading 19 designed to pierce the metatarsal head (c), in order to make the implant integral with the metatarsal component (d).

As can be seen in figure 9, the longitudinal axis of the pin forms an angle of more than 90° with respect to the axis of the screw.

In the median zone of the paddle-shaped part 16, that is in the part between the threaded hole 17 and the base of the paddle-shaped part, there are two parallel non-threaded holes 20 and 20', which are angled with a direct convergence towards the tip of the screw 12.

These holes are designed to accommodate two temporary rigid metal wires 21 and 22, acting as Joy Sticks, used to adjust the pin on the axial and frontal planes of the metatarsal head ("□" e "□", alpha and omega) once inserted in the metatarsal head (c).

The flat-head screw 12, inserted in the threaded hole 17 of the paddle-shaped part 16 of the curved pin, and subsequently screwed into the metatarsal head (c), is designed to block the metatarsal bone component (d) to the metatarsal head (c) (see figure 9).

As already stated, the objective of the implant is to fix the two bone fragments (c, d) following osteotomy of the first metatarsal in hallux valgus deformities, to adjust its lateralization, plantarization and orientation of the frontal and sagittal planes (alpha and omega), preventing mobilization between the two fragments (c, d) during bone consolidation and allowing bone union between the two fragments without their displacement.

From the procedural point of view, as can be seen in figure 9, after osteotomy of the first metatarsal (m), the curved pin implant 10, appropriately mounted on the positioning guide and blocked by a threaded cannula which also acts as a perforation guide for the preparation of the housing for the flat-head screw 12, is inserted in the medullary canal (a) of the metatarsal bone segment (d) at its base (e).

The insertion of the pin must be carried out with the exterior 15 of the curved part 14 resting against the external cortex (b) and with the axis X of the paddle-shaped part 16 axial to the metatarsal head (c).

In this phase, the curved pin 10 thus inserted automatically causes the translation (h) of the metatarsal head (c) with respect to the metatarsal bone segment (d), as can be seen in figure 9.

The first rigid metal wire 21 can now be inserted in the first convergent hole 20 positioned medially to the hole 17 accommodating the screw 12. By piercing the metatarsal head (c), this wire makes it possible to manually adjust the metatarsal head and to correct it on the sagittal, frontal and medial planes (alpha and omega).

The positioning of the second rigid metal wire 21a in the second convergent hole 20a, medial to the hole accommodating the screw 12, makes it possible to temporarily block the relationship between the two bone segments (c, d) in the required position.

The housing for the implant blocking screw is then prepared, consisting of perforation of the metatarsal head (c) with a graduated drill bit, through the guide cannula.

The length of the screw 12 to be used is determined on the basis of the depth of the perforation made, which can be read on the graduated scale of the perforating drill.

The screw 12 which will stabilize the implant 10 between the metatarsal head (c) and the metatarsal bone segment (d) is then inserted after removing the guide cannula.

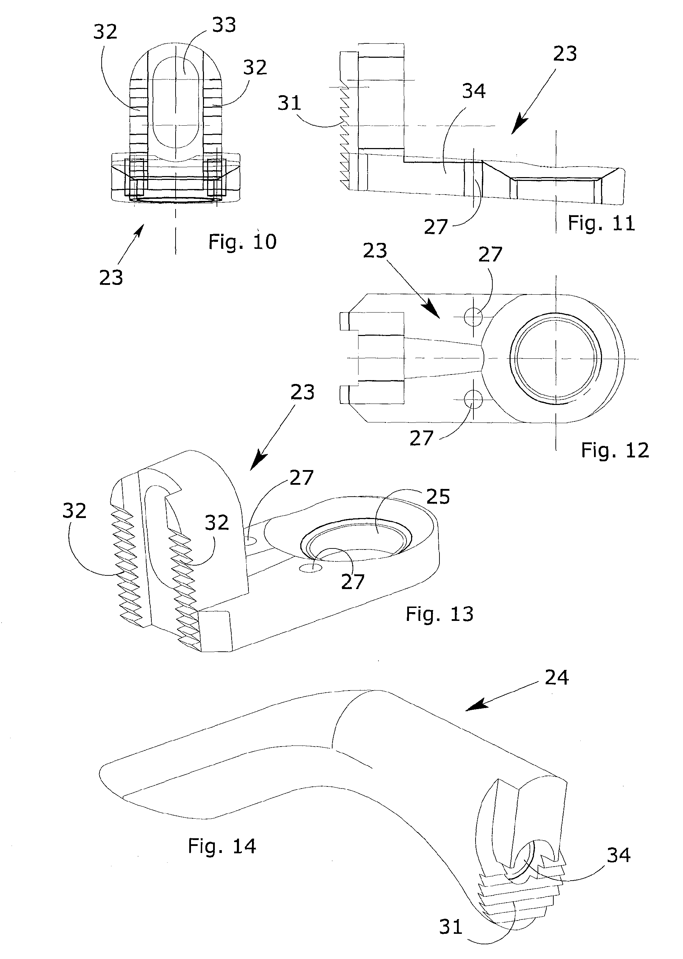

Figures 10 to 20 show another embodiment of the implant.

The main objective of this further embodiment is to fix the two bone segments of the first metatarsal after distal osteotomy, in hallux valgus, and, according to the anatomical requirement, to increase the lateralization, adjust the plantarization and orientation on the frontal and sagittal planes, and to prevent mobilization between the two bone portions, thus allowing bone union without displacement.

The modular implant according to the invention, which substantially consists of two components, an L-shaped plate 23 angled at about 3° with respect to the longitudinal axis of the assembled implant and a cone-shaped component 24 with a curved tip.

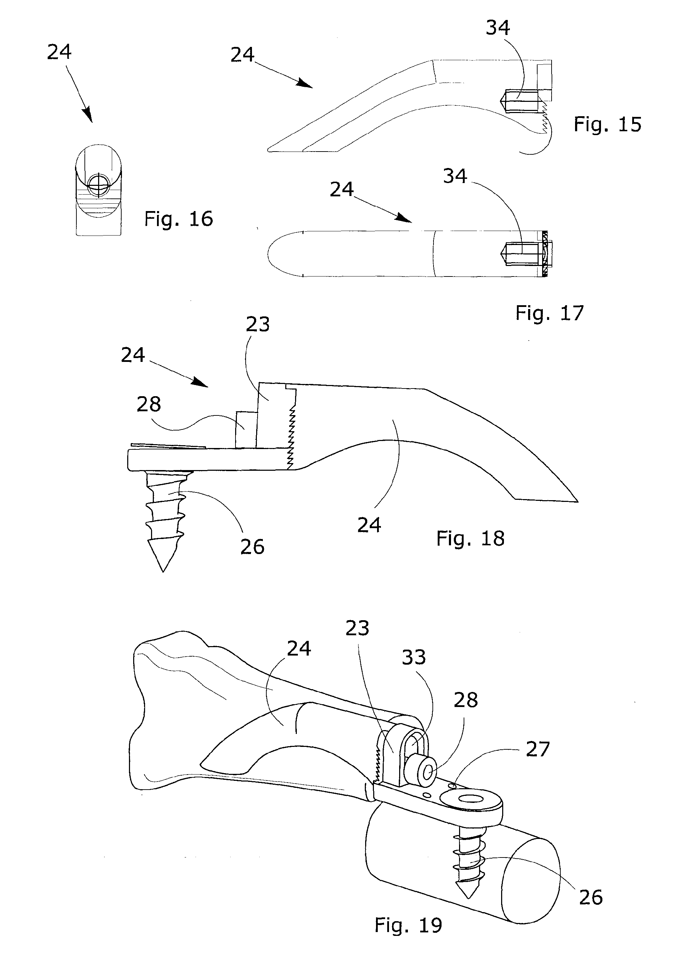

The plate 23 is fitted with a hole 25 flared at the surface and threaded in the lower part to accommodate a flat-head screw 26 on one side, two micro holes 27 below for the pre-stabilization and a micro grub screw 28 for blocking the two components when translation is complete, as can be seen in figures 18 and 19.

The use of an instrument 29, visible in figures 21 and 22, is also foreseen for positioning of the endosseous implant, and a translator instrument 30 to be mounted on the modular endo-osseous implant, with a thrust system to produce the millimetric translation.

The base of the cone 24 has a knurled section 31 designed to couple with a respective knurled section 32 of the plate 23.

The plate 23 is in turn equipped with a slot 33 for the blocking grub screw 28, which is inserted in the threaded hole 34 of the cone 24.

The cone-shaped component 24 with the curved tip is sunk into the metatarsal medullary canal as far as the base of the cone, close to the knurled section 32, where it joins with the base of the L-shaped plate 23, at its knurled section 32, which will protrude from the line of the osteotomy of the metatarsal segment.

The L-shaped plate 23 will accommodate the micro grub screw 28, which holds the two implant components 23 and 24 together and, once screwed in, stabilizes them.

Loosening the grub screw 28 allows the lateral translation of the L-shaped plate 23 with respect to the axis of the cone-shaped component 24 with the curved tip.

Tightening the grub screw 28 makes it possible to stabilize the lateral translation of the L-shaped plate 23 with respect to the axis of the cone-shaped component 24, by means of the special translator instrument 30, mounted on the base 34 of the L-shaped plate 23.

The positioning instrument 29 consists of an angled cylindrical grip 35 with the implant assembly housing on the side of the L-shaped plate, and it works in association with a threaded blocking cannula for preparation of the hole for the stabilization screw 26 of the endo-osseous implant.

The translator instrument 30 consists of a parallelepiped thrust block 36 with two sliding pins 37 underneath which are fixed to a base 38 from which they move.

This base 38 is also equipped with a thrust rod 39, passing medially through the base 38, and ending with a threaded rod 40 at the distal end and which, when screwed down by means of its knob 41, produces the movement between the two modular components 23 and 24, thus achieving the correct alignment between the metatarsal head and the metatarsal segment of the first metatarsal in relation to the anatomical requirement.

It can be noted that the implant according to the invention described and illustrated here makes it possible to achieve the following aims:
• to perform the surgical procedure easily and with precision;
• to adjust the bone fragment (c) of the first metatarsal with respect to the metatarsal bone fragment (d) after osteotomy (m) according to the anatomical requirement of each patient;
• to provide the implant with great stability in relation to the two metatarsal bone segments (c, d).

In addition, as already stated, the aim of the implant is also to fix the two bone segments after distal osteotomy of the first metatarsal in hallux valgus deformities, and to subsequently adjust the progressive lateralization using the special translator instrument 30, and to stabilize the implant of the bone components, allowing bone consolidation, in the correct position of the translation necessary for the anatomical requirement.

From a procedural point of view, after distal osteotomy of the first metatarsal, the endo-osseous implant is sunk into the medullary canal of the first metatarsal. Insertion of the modular implant, consisting of the cone-shaped component with a curved tip 24 and the L-shaped plate 23, assembled and mounted on the positioning instrument, and fixed together by means of the special cannulated fixing cylinder, must be performed with the flat side of the end of the cone 24 resting against the lateral cortex and the L-shaped plate 23 thus resting against the metatarsal head.

During this phase of the procedure, the modular implant causes the automatic translation of the metatarsal head with respect to the metatarsal bone segment. The metatarsal head is then pre-stabilized with respect to the L-shaped plate 23 by inserting the two small metal wires in the micro holes 27 below the hole 25 for the endo-osseous fixing screw 26.

The housing is then prepared for the endo-osseous implant stabilization screw 26, using a drill to perforate the cannulated cylinder followed by insertion and screwing in of the bone screw.

The positioning instrument 29 is then removed, the L-shaped plate 23 of the endo-osseous implant is fixed to the metatarsal head and the translation is corrected by means of the translator instrument 30 appropriately mounted on the L-shaped plate 23.

The assembly of the two modular components 23 and 24 is then loosened by unscrewing the micro grub screw 28. Translation is achieved by turning the knob 41 of the rod 39, checking the gradual movement between the curved tip cone 24 and the L-shaped plate 23 which will produce the correct translation between the two bone components according to the anatomical requirement, until maximum translation of the implant is achieved.

When the correction is complete, the two components 23 and 24 are then definitively blocked together by tightening the grub screw 28, and the endo-osseous implant thus becomes a one-piece body.

The invention is described above with reference to a preferred embodiment. It is nevertheless clear that the invention is susceptible to numerous variations which lie within its scope, within the framework of technical equivalents.

## Claims

1. An implant for fixing two bone fragments together, following the axial correction of the metatarsal head with respect to the metatarsal segment in hallux valgus deformities, in osteotomy procedures in general, **characterised in that** it consists of a cylindrical cross-section curved pin (10), with a tip (15) of the curvature (14) and a proximal part in the shape of a paddle (16) equipped with a central hole flared at the surface and threaded in its lower portion, designed to accommodate a flat-head screw (12) which prevents dangerous protruberances into the soft tissues.

2. An implant for fixing two bone fragments together according to the foregoing claim, **characterised in that** the cylindrical cross-section curved pin (10) presents a tip (15) of the curvature (14) which rests against the external cortex (b) of the metatarsal bone (d), and a distal bevelled part (13) which rests against and/or is positioned at the opposite distal base (e) of the medullary canal of the metatarsal bone (a).

3. An implant for fixing two bone fragments together according to either of the foregoing claims, **characterised in that** the paddle-shaped part (16) has two small holes (20, 20') in the part proximal to the central hole (17), the axis of these small holes being convergent on the longitudinal axis of the central hole (17), to accommodate two temporary rigid metal wires (21, 22).

4. An implant for fixing two bone fragments together according to any of the foregoing claims, **characterised in that** two temporary rigid metal wires (21, 22) are inserted in the two holes (20, 20') and act as Joy Sticks to adjust the pin on the axial and frontal planes of the metatarsal head ("□" e "□", alpha and omega) once inserted in the metatarsal head (c).

5. An implant for fixing two bone fragments together according to any of the foregoing claims, **characterised in that** the cylindrical curved pin (10) is sunk into the medullary canal (a) of the metatarsal portion and the paddle-shaped part (16) projecting from the osteotomy line rests against the osteotomized metatarsal head (c), automatically causing medial translation.

6. An implant for fixing two bone fragments together according to any of the foregoing claims, **characterised in that** the flat-head flared screw (12) of the implant, which prevents damage to the soft tissues, has mechanical type threading (18) below the flared head to allow stabilization with the threaded hole (17) in the paddle-shaped part (16) of the cylindrical curved pin and bone type threading (19) below the mechanical type threading (18) for stabilization of the metatarsal head bone with the implant.

7. An implant for fixing two bone fragments together according to any of the foregoing claims, **characterised in that** the screw (12) presents a "trocar" or pyramidal shaped tip.

8. An implant for fixing two bone fragments together according to any of the foregoing claims, **characterised in that** the longitudinal axis of the pin forms an angle of more than 90° with respect to the axis of the screw.

9. An implant for fixing two bone fragments together according to any of the foregoing claims, **characterised in that** another embodiment of the modular implant substantially consists of two components, an L-shaped plate (23) angled at approximately 3° with respect to the longitudinal axis of the assembled implant, and a cone-shaped component (24) with a curved tip.

10. An implant for fixing two bone fragments together according to any of the foregoing claims, **characterised in that** the plate (23) is fitted with a hole (25) flared at the surface and threaded in its lower portion to accommodate a flat-head screw (26) on one side, two micro holes (27) below for the pre-stabilization and a micro grub screw (28) for blocking the two modular components when translation is complete.

11. An implant for fixing two bone fragments together according to any of the foregoing claims, **characterised in that** it foresees the use of an instrument (29), for positioning the endo-osseous implant, and a translator instrument (30) to be mounted on the endo-osseous implant, with a thrust block to produce the millimetric translation.

12. An implant for fixing two bone fragments together according to any of the foregoing claims, **characterised in that** the base of the cone-shaped component (24) is equipped with a knurled section (31) designed to couple with a respective knurled section (32) on the plate (23).

13. An implant for fixing two bone fragments together according to any of the foregoing claims, **characterised in that** the plate (23) is in turn equipped with a slot (33) to accommodate the blocking grub screw (28), which is then inserted in the threaded housing (34) of the cone-shaped component (24).

14. An implant for fixing two bone fragments together according to any of the foregoing claims, **characterised in that** the cone-shaped component (24) with the curved tip is sunk into the metatarsal medullary canal as far as the base of the cone, close to the knurled section (32), where it joins with the base of the L-shaped plate (23), at the knurled section (32), which will protrude from the line of the osteotomy of the metatarsal segment.

15. An implant for fixing two bone fragments together according to any of the foregoing claims, **characterised in that** the L-shaped plate (23) accommodates the micro grub screw (28), which holds the two implant components (23, 24) together and which, once tightened, stabilizes them.

16. An implant for fixing two bone fragments together according to any of the foregoing claims, **characterised in that** loosening the grub screw (28) allows the lateral translation of the L-shaped plate (23) with respect to the axis of the cone-shaped component (24) with a curved tip.

17. An implant for fixing two bone fragments together according to any of the foregoing claims, **characterised in that** tightening the grub screw (28) makes it possible to stabilize the lateral translation of the L-shaped plate (23) with respect to the axis of the cone-shaped component (24), by using the special translator instrument (30), mounted on the base (34) of the L-shaped plate (23).

18. An implant for fixing two bone fragments together according to any of the foregoing claims, **characterised in that** the positioning instrument (29) consists of an angled cylindrical grip (35) with the implant assembly housing on the side of the L-shaped plate (23), and it works in association with a threaded blocking cannula for preparation of the hole for the stabilization screw (26) of the endo-osseous implant.

19. An implant for fixing two bone fragments together according to any of the foregoing claims, **characterised in that** the translator instrument (30) consists of a parallelepiped thrust block (36) with two sliding pins (37) underneath which are fixed to a base (38) from which they move.

20. An implant for fixing two bone fragments together according to any of the foregoing claims, **characterised in that** the base (38) is equipped with a thrust rod (39), passing medially through the base (38), and ending with a threaded rod (40) at the distal end and which, when screwed down by means of its knob (41), produces the movement between the two modular components (23, 24), thus achieving the correct alignment between the metatarsal head and the metatarsal segment of the first metatarsal in relation to the anatomical requirement.
